# EUROPEAN PATENT SPECIFICATION

(11) **EP 0 351 127 B1**
(45) Date of publication and mention of the grant of the patent: **22.12.1993**
(21) Application number: 89306826.2
(22) Date of filing: 05.07.1989
(51) Int. Cl.: C07C 231/24, C12P 13/14

(54) **Method of purifying L-glutamine**
Verfahren zur Reinigung von L-Glutamin
Procédé pour la purification de L-Glutamine

(30) Priority: 11.07.1988 JP 170962/88
(43) Date of publication of application: 17.01.1990
(73) Proprietor: AJINOMOTO CO., INC., Tokyo 104 (JP)
(72) Inventor: Miyazawa, Masashi Kawasaki Factory, Kawasaki-shi Kanagawa-ken (JP); Kaneko, Toyokazu Kawasaki Factory, Kawasaki-shi Kanagawa-ken (JP); Kaneko, Tetsuya Kawasaki Factory, Kawasaki-shi Kanagawa-ken (JP); Yarita, Kenichi Kawasaki Factory, Kawasaki-shi Kanagawa-ken (JP)
(74) Representative: Bond, Bentley George

(56) References cited:
- FR-A- 2 619 380
- GB-A- 1 543 765
- US-A- 3 420 744
- CHEMICAL ABSTRACTS, vol. 83, 24th November 1975, abstract no. 176707p, Columbus, Ohio, US; & JP-A-75 95 481

## Description

This invention relates to a method for purifying L-Glutamine (L-Gln) which is used as the starting material for medicines and for other purposes.

L-glutamine can be prepared by organic synthesis, but the fermentation process for its preparation has been improved in recent years, and can produce this compound at high efficiency. L-glutamine tends to undergo decomposition under certain temperature and pH conditions to form pyrrolidonecarboxylic acid (PCA), and once PCA is formed, it is practically impossible to restore L-glutamine from this decompositon product. Hence, scrupulous care must be taken in purifying this compound.

The so-called "resin-treatment process" has been generally used for isolating L-glutamine from its fermentation liquor, in which the liquor is directly, or after removal of microbial cells by a separator of centrifugal or filtration type, brought into contact with a strongly acidic cation-exchange resin to absorb L-glutamine, followed by elution with an alkaline solution and crystallization. This is an excellent process for isolating and purifying L-glutamine because most of the impurities contained in its fermentation liquor, such as foreign amino acids, PCA (a decomposition product of L-glutamine), microbial cells, soluble proteins, inorganic salts and sugars left unconsumed, can be removed unadsorbed.

This conventional process, however, suffers from problems of industrial importance, for example, much water is necessary for reactivation and washing of the ion-exchange resin, imposing a big burden of waste water treatment, and chemical decomposition of L-glutamine is liable to occur as a result of contact with acids and alkalis resulting in a low recovery rate from the fermentation liquor.

The "direct crystallization process", in which crystals of L-Gln are directly recovered from its fermentation liquor without any pretreatment, generally gives crystals of lower purity compared with the "resin-treatment process". Hence, establishing a method of effectively purifying low-purity crystals of L-glutamine is an essential factor to make the "direct crystallization process" sufficiently practical.

As a result of various examinations of the impurities in the low-purity L-glutamine obtained from its fermentation liquor through crystallization by concentration or cooling, it was found that foreign amino acids (fermentation byproducts), PCA (a decomposition product of L-glutamine), inorganic salts, proteins and sugars are major components of the impurities. Further intensive studies on how to remove these impurities have led us to establish an effective purification method using anion-exchange resins. L-glutamine of low purity has hitherto been purified by recrystallization. In this conventional method, L-glutamic acid and PCA tend to be incorporated in recrystallized products, and hence recrystallization must be repeated to obtain high-purity products, thus making its practical application almost impossible in terms of cost. Also as a result of intensive studies to alleviate the burden of waste water treatment and to achieve higher recovery rate of high-quality L-glutamine from its fermentation liquor using the "resin-treatment process", we have found that these objects can be achieved if the crystals of L-glutamine obtained by concentration or direct cooling of its fermentation liquor are treated with an activated anion-exchange resin.

Furthermore, with the method of the present invention L-glutamic acid and PCA, as well as inorganic salts, can be completely removed by adsorption on an anion-exchange resin, significantly enhancing the quality of final products. The problems involved in the "direct crystallization method" thus can be solved completely.

According to the present invention there is provided a method of purifying L-glutamine which comprises (1) obtaining a slurry of crystals of L-glutamine by concentrating or cooling a fermentation liquor, or a solution obtained by filtering off microbial cells from a fermentation liquor; (2) where necessary concentrating the slurry of crystals to approximately 70 g/I or higher; (3) separating the crystals from the fermentation liquor and forming an aqueous solution of them in water; and (4) contacting the aqueous solution with an OH-type anion-exchange resin to remove impurities from the solution by adsorption.

The fermentation liquor used in the method of this invention preferably contains 40 g/I or more of L-glutamine. Use of a liquor containing less than 40 g/I L-glutamine is less preferred because of the low crystallization rate and the low purity of formed crystals. Prior to crystallization, the fermentation liquor may be subjected to any pretreatment that results in little or no recovery of L-glutamine, for example, the removal of microbial cells.

For step (1) the fermentation liquor, or a solution obtained therefrom by filtering off microbial cells, is preferably concentrated or cooled at pH 5.6±0.5 to crystalize out L-glutamine. pH adjustment is carried out by addition of an acid (e.g., hydrochloric acid) because of the pH value of fermentation liquor is generally about 7.0. It is preferable that the pH adjustment be carried out prior to concentration or cooling, but it may be done during concentration or cooling when required. If the slurry of crystals is obtained by concentration it is continued, or, if the slurry of crystals is obtained by cooling, concentration is carried out after cooling, until the content of L-glutamine reaches a level of 70 g/I or higher which ensures a suitable crystallization rate. Step (2) is unnecessary in those cases where the fermentation liquor contains 70 g/I or more of L-glutamine. Cooling for crystallization may be conducted at a suitable temperature in the range of 2 to 30 _{°} C, which may vary with the properties of fermentation liquor being treated, the predetermined crystallization rate and other facts. Crystallization by cooling may not be suitable in some cases due to the properties of the fermentation liquor. Also where equipment capable of the continuous concentration and separation of formed crystals is available crystallization by concentration is preferred to crystallization by cooling. By suitable crystallization rate it is meant such a rate that the formed crystals, when treated with an anion-exchange resin and then purified by commonly used techniques, show a predetermined purity. This value may vary with the properties of the fermentation liquor being treated, the intended purity level of the final products, the type of crystallizer and other equipment used, the crystallization conditions and other factors.

The slurry obtained by crystallization, after concentration if necessary, is separated into crystals and mother liquor, and the crystals are normally washed one to several times with a small amount of water. A solid-liquid separator may be used, for example, a centrifuge of the batch or continuous type.

The collected crystals are then brought into contact with an OH-type anion-exchange resin in the form of an aqueous solution. The resin used may be a strongly basic anion-exchange resin (such as IRA-430 and IRA-904 of Rohm & Haas, and PA-416 and SA-21A of Mitsubishi Chemical Industries), or preferably a moderately or weakly basic anion-exchange resin (such as IRA-35, IRA-45, IRA-68 and IRA-93 of Rhom & Haas, and WA-10, WA-21 and WA-30 of Mitsubishi Chemical Industries). A suitable amount of resin to be used is such that required amounts of impurities in the solution of crystals can be removed, which depends of the intended purity of final products. This amount may be determined by taking, as an index, a proper type of impurity, such as glutamic acid and PCA. The resin may be used mixing it with a solution of L-glutamine in a bath, but use of the commonly employed column system (both single-column and multi- column systems) ensures a more simple operation.

In order to minimize the decomposition of L-glutamine, it is preferable that its aqueous solution be brought into contact with the resin at a temperature of about 0 to 40 _{°} C and at a pH of about 5.6±0.5.

The aqueous solution of L-glutamine from which impurities have been removed by contact with an anion-exchange resin may then be purified by unusual methods (for example, decolourization with activated charcoal of the like, followed by concentration, as required, and crystallization by cooling), thus giving final products.

The mother liquor left after the crystals of L-glutamine have been recovered from its fermentation liquor by concentration or cooling may still contain L-glutamine in an amount which corresponds to its solubility in water, in addition to containing impurities such as L-glutamine acid, PCA, microbial cells, soluble proteins, inorganic salts and sugars. It is therefore preferable to recover any L-glutamine left in the mother liquor to enhance its recovery rate. This recovery can be effected by first removing polymeric substances which inhibit crystal growth (such as soluble proteins and microbial cells) by the known ultrafiltration (UF) membrane technique, and then isolating L-glutamine crystals by usual methods, such as crystallization by concentration or cooling. The crystals thus obtained are purified by treatment with an anion-exchange resin in the same way as described above, either alone or in combination with the crystals separated above from the fermentation liquor through crystallization by concentration or cooling. Thus, removal of crystal-growth inhibiting substances from the mother liquor enables isolation of high-purity L-glutamine crystals and enhances the total yield of final products, thus providing a purification method of high practical utility. The present invention is illustrated, but in no way limited by the following Examples.

### Example 1

Fifty litres of a fermentation liquor containing 42 g/I L-Gln was adjusted to pH 5.6 by addition of 35%-HCI, concentrated to a concentration of 120 g/I and then cooled to 5 _{°} C at a speed of 5 _{°} C/hr to effect crystallization. The slurry thus obtained was separated into L-Gln crystals ( No.1 ) and mother liquor containing microbial cells and other impurities by using a superdecanter P4Y.

The separated mother liquor ( 48 litres ) was heated and filtered through hollow-fiber UF membrane ( nominal limit N.W.: 6000 ) to remove microbial cells and other impurities, and the transparent filtrate thus obtained was concentrated to effect crystallization (final L-Gln concentration: 230 g/I ).

The concentrated slurry was transferred to a crystallizer, cooled with stirring from 45 _{°} C down to 20 _{°} C, and put into a basket-type centrifugal separator to remove mother liquor. The crystal cake collected in the basket was washed with water in an amount of about 40% of its weight, giving L-Gln crystals ( No.2 ).

The amounts of L-glutamine recovered ( No.1 and No.2 ) were 1.2 Kg and 0.6 Kg, repectively, the total recovery rate from the fermentation liquor amounting to 85.7%.

A mixture of 0.6 Kg L-Gln No.1 crystals and 0.3 Kg No.2 crystals was dissolved in water by heating to 40 _{°} C, preparing an aqueous solution containing 40 g/ 1 L-Gln ( pH 4.8 ). This solution was allowed to pass through a column packed with 0.5 liter of Amberlite IRA-68 [OH]-type ( a moderately basic anion-exchange resin ) to remove L-glutamic acid (L-Glu ), PCA, SO_{4̅} and other impurities by adsorption. The withdrawn solution was decolorized with 45 g activated charcoal at 40 _{°} C, the decolorized solution was filtered through a suction filter, the filtrate (23 litres ) was concentrated to effect crystallization, and the resulting slurry was cooled, giving 0.8 Kg of L-Gln pure crystals ( recovery rate from fermentation liquor: 76% ).

The contents of impurities in L-Gln crystals obtained in respective purification stages are shown in the table below.

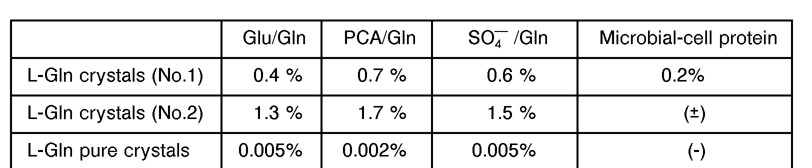

### Example 2

Fifty liters of a fermentation liquor containing 74 g/I L-Gln was adjusted to pH 5.6 by addition of 35%-HCI, cooled to 5 °C at a speed of 5 _{°} C/hr in a crystallizer fitted with a stirrer, and then matured at 5 °C for four hours to effect crystallization. The slurry thus obtained was treated in a superdecanter P4Y, giving L-Gln crystals (No.1).

The separated mother liquor was treated in the same manner as in Example 1, giving L-Gln crystals ( No.2 ).

The amounts of L-glutamine recovered (No.1 and No.2 ) were 1.5 Kg and 1.6 Kg, respectively, the total recovery rate from the fermentation liquor amounting to 83.8%.

A mixture of L-Gln No.1 crystals and No.2 crystals was dissolved in water in the same way as in Example 1, the solution was allowed to pass through a column packed with the moderately basic anion-exchange resin, followed by decolorization, concentration and cooling to effect crystallization, giving 2.7 Kg of L-Gln pure crystals ( recovery rate from fermentation liquor: 73% ).

The contents of impurities in L-Gln crystals obtained in respective purification stages are shown in the table below.

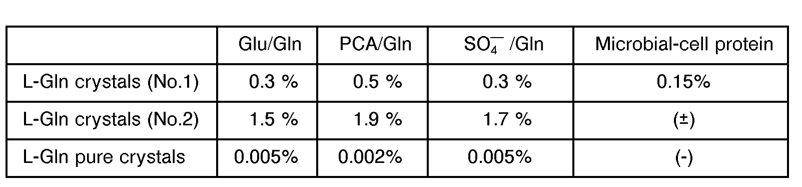

### Comparative Example 1

A mixture of 0.6 g Kg L-Gln crystals (No.1) and 0.3 Kg crystals (No.2), obtained in Example 1, was recrystallized in a usual way as described below. The crystals were dissolved in water to a concentration of 40 g/I, activated charcoal was added to the solution in an amount of 5% based on the weight of L-Gln, and decolorization was conducted by heating at 40 °C. After filtering off the activated charcoal, the decolorized solution was concentrated to effect crystallization, and the resulting slurry was cooled to 5°C, giving 0.81 Kg of pure crystals ( recovery rate from fermentation liquor: 77% ).

The contents of impurities in L-Gln crystals obtained in respective purification stages are shown in the table below.

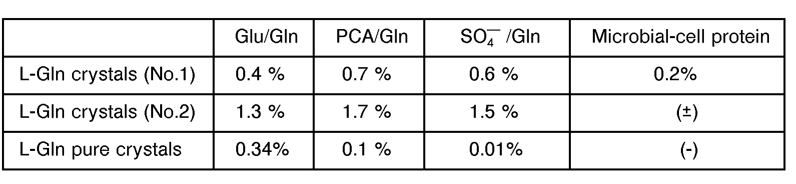

### Comparative Example 2

Fifty liters of a fermentation liquor containing 42 g/I L-Gln were freed from microbial cells by using a centrifuge, and the treated solution was adjusted to pH 1.8 and allowed to pass through a column packed with 50 liters of Duolite C-20 ( a strongly acidic cation-exchange resin ) to absorb L-Gln on the resin. In this operation, PCA, soluble proteins, inorganic salts, sugars and part of Glu were removed unadsorbed.

After washing the columb with water, the absorbed L-Gln was eluted with 0.5N-NH₄0H, the elutant ( 65 liters ) was concentrated to a concentration of 450 g/I, and the resulting slurry was cooled to 5 _{°} C and treated in a basket-type centrifugal separator, giving 1.6 Kg of L-Gln crystals (No.1) ( recovery rate: 76% ). The mother liquor was further concentrated and cooled, but no crystals of L-Gln were separated out. The crystals (No.1) obtained above were purified by recrystallization in the same way as in Comparative Example 1, affording 1.44 Kg of pure crystals ( recovery rate from fermentation liquor: 68.6 ).

The contents of impurities in L-gln crystals obtained in respective purification stages are shown in the table below.

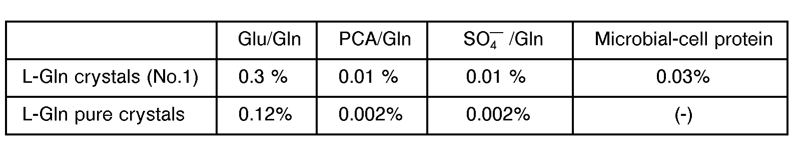

The method of the present invention enhances the recovery rate of L-glutamine from its fermentation liquor, minimizes incorporation of impurities, particularly glutamic acid, thereby giving high-purity final products, and significantly reduces the amount of water to be used.

## Claims

1. A method of purifying L-glutamine which comprises the following steps:
(1) obtaining a slurry of crystals of L-glutamine by concentration or cooling a fermentation liquor, or a solution obtained by filtering off microbial cells from a fermentation liquor;
(2) where necessary concentrating the slurry of crystals to approximately 70 g/I or higher;
(3) separating the crystals from the fermentation liquor or filtered solution and forming an aqueous solution of them in water; and
(4) contacting the aqueous solution with an OH-type anion-exchange resin to remove impurities from the solution by adsorption.

2. A method according to claim 1, wherein the fermentation liquor contains approximately 40 g/I or more of L-glutamine.

3. A method according to claim 1 or 2, wherein in step (1) the slurry of crystals is obtained by cooling the fermentation liquor or filtered solution at a temperature in the range of 2 to 30 _{°} C.

4. A method according to claim 1, 2 or 3, wherein step (1) is carried out at a pH of approximately 5.6±0.5.

5. A method according to any preceding claim, wherein the anion-exchange resin is a strongly basic anion-exchange resin.

6. A method according to any one of claims 1 to 4, wherein the anion-exchange resin is a moderately or weakly basic anion-exchange resin.

7. A method according to any preceding claim, wherein step (4) is carried out at a temperature in the range of 0 to 40 _{°} C and at a pH of approximately 5.6±0.5.

8. A method according to any preceding claim, which further comprises the following steps:
(5) purifying the solution obtained from step (4) using standard techniques; and
(6) crystallization of the purified solution of step (5) by concentration or cooling.

9. A method according to claim 8, wherein the purification of step (5) is carried out using activated charcoal.

10. A method according to any preceding claim, wherein the fermentation liquor is treated, for example by ultrafiltration, to remove polymeric substances which inhibit crystal growth prior to step (1).

## Patentansprüche

1. Verfahren zum Reinigen von L-Glutamin, welches die folgenden Schritte umfaßt:
(1) Erhalten einer Aufschlämmung von L-Glutamin-Kristallen durch Konzentrieren oder Kühlen einer Fermentationsbrühe oder einer durch Abfiltrieren von Mikrobenzellen aus einer Fermentationsbrühe erhaltenen Lösung,
(2) erforderlichenfalls Konzentrieren der Aufschlämmung der Kristalle bis zu 70 g/I oder mehr,
(3) Abtrennen der Kristalle aus der Fermentationsbrühe oder der filtrierten Lösung und Ausbilden einer wässrigen Lösung dieser Kristalle in Wasser, und
(4) In Kontakt bringen der wässrigen Lösung mit einem Anionenaustauscherharz in der OH-Form, um Verunreinigungen aus der Lösung durch Adsorption zu entfernen.

2. Verfahren gemäß Anspruch 1, worin die Fermentationsbrühe etwa 40 g/I oder mehr L-Glutamin enthält.

3. Verfahren gemäß Anspruch 1 oder 2, worin in Schritt (1) die Aufschlämmung der Kristalle durch Abkühlen der Fermentationsbrühe oder der filtrierten Lösung auf eine Temperatur im Bereich von 2 bis 30 ° c erhalten wird.

4. Verfahren gemäß Anspruch 1, 2 oder 3, worin Schritt (1) bei einem pH von ungefähr 5,6±0,5 durchgeführt wird.

5. Verfahren gemäß einem der vorhergehenden Patentansprüche, worin das Anionenaustauscherharz ein stark basisches Anionenaustauscherharz ist.

6. Verfahren gemäß einem der Ansprüche 1 bis 4, worin das Anionenaustauscherharz ein mäßig- oder schwach-basisches Anionenaustauscherharz ist.

7. Verfahren gemäß einem der vorhergehenden Patentansprüche, worin Schritt (4) bei einer Temperatur im Bereich von 0 bis 40 _{°} C und bei einem pH von ungefähr 5,6 ± 0,5 durchgeführt wird.

8. Verfahren gemäß irgend einem vorhergehenden Patentanspruch, welches zusätzlich die folgenden Schritte umfaßt:
(5) Reinigen der aus Schritt (4) erhaltenen Lösung mit Hilfe von Standard-Methoden und
(6) Kristallisieren der gereinigten Lösung des Schrittes (5) durch Konzentrieren oder Kühlen.

9. Verfahren gemäß Anspruch 8, worin die Reinigung in Schritt(5) unter Verwendung von Aktivkohle durchgeführt wird.

10. Verfahren gemäß irgend einem vorhergehenden Patentanspruch, worin die Fermentationsbrühe vor dem Schritt (1) zur Entfernung von polymeren Substanzen, welche das Kristallwachstum inhibieren, behandelt wird, beispielsweise durch Ultrafiltration.

## Revendications

1. Méthode de purification de la L-glutamine qui comprend les étapes suivantes :
(1) l'obtention d'une pâte de cristaux de L-glutainine par concentration ou refroidissement d'une liqueur de fermentation, ou d'une solution obtenue par filtration pour éliminer les cellules microbiennes d'une liqueur de fermentation;
(2) si nécessaire, la concentration de la pâte de cristaux à environ 70 g/I ou plus ;
(3) la séparation des cristaux de la liqueur de fermentation ou de la solution filtrée et la formation d'une solution aqueuse de cristaux dans l'eau et
(4) la mise en contact de la solution aqueuse avec une résine échangeuse d'anions du type OH pour éliminer les impuretés de la solution par adsorption.

2. Méthode selon la revendication 1, dans laquelle la liqueur de fermentation contient environ 40 g/I ou plus de L-glutamine.

3. Méthode selon la revendication 1 ou 2, dans laquelle dans l'étape (1) la pâte de cristaux est obtenue par refroidissement de la liqueur de fermentation ou de la solution filtrée à une température située dans l'intervalle de 2 à 30 _{°} C.

4. Méthode selon la revendication 1, 2 ou 3, dans laquelle l'étape (1) est conduite à un pH d'environ 5,6 ± 0,5.

5. Méthode selon une quelconque des revendications précédentes, dans laquelle la résine échangeuse d'anions est une résine échangeuse d'anions très basique.

6. Méthode selon une quelconque des revendications 1 à 4, dans laquelle la résine échangeuse d'anions est une résine échangeuse d'anions modérément ou faiblement basique.

7. Méthode selon une quelconque des revendications précédentes, dans laquelle l'étape (4) est conduite à une température située dans l'intervalle de 0 à 40 _{°} C et à un pH d'environ 5,6 ± 0,5.

8. Méthode selon une quelconque des revendications précédentes, qui comprend en outre les étapes suivantes :
(5) purification de la solution obtenue dans l'étape (4) par les techniques standards ; et
(6) cristallisation de la solution purifiée de l'étape (5) par concentration ou refroidissement.

9. Méthode selon la revendication 8, dans laquelle la purification de l'étape (5) est conduite en utilisant du charbon activé.

10. Méthode selon une quelconque des revendications précédentes, dans laquelle la liqueur de fermentation est traitée, par exemple par ultrafiltration, pour éliminer les substances polymères qui inhibent la croissance des cristaux avant l'étape (1).
